(19) Europäisches Patentamt

European Patent Office

Office européen des brevets

(11) **EP 1 656 916 A1**

(12) **DEMANDE DE BREVET EUROPEEN**

(43) Date de publication:
**17.05.2006 Bulletin 2006/20**

(51) Int Cl.:
*A61F 13/04* *(2006.01)*    *A61F 13/06* *(2006.01)*
*A61F 13/10* *(2006.01)*    *A61F 13/08* *(2006.01)*

(21) Numéro de dépôt: **04447247.0**

(22) Date de dépôt: **10.11.2004**

(84) Etats contractants désignés:
**AT BE BG CH CY CZ DE DK EE ES FI FR GB GR
HU IE IS IT LI LU MC NL PL PT RO SE SI SK TR**
Etats d'extension désignés:
**AL HR LT LV MK YU**

(71) Demandeur: **Université Libre de Bruxelles
1050 Brussels (BE)**

(72) Inventeurs:
• **Schuind, Frédéric
3220 Holsbeek (BE)**

• **Liegeois, Jean-Maire
4654 Charneux (BE)**
• **Dejaie, Laurent
4000 Liège (BE)**
• **Gerday, Jonathan
4560 Clavier (BE)**
• **Moulart, Françoise
1070 Bruxelles (BE)**

(74) Mandataire: **Van Malderen, Joelle et al
pronovem - Office Van Malderen
Avenue Josse Goffin 158
1082 Bruxelles (BE)**

(54) **Elément tubulaire pour contention orthopedique**

(57)    La présente invention se rapporte à un élément tubulaire pour la réalisation d'une contention orthopédique, comportant au moins un textile élastique servant de support d'imprégnation à un produit d'imprégnation, ledit produit d'imprégnation comportant une résine de polyuréthanne, dans lequel ledit élément tubulaire est essentiellement étirable et rétractable de manière réversible par retour élastique dudit support d'imprégnation, dans un plan perpendiculaire à l'axe longitudinal dudit élément tubulaire lorsque ladite résine de polyuréthanne est à une température supérieure à sa température de ramollissement et avant recristallisation de celle-ci, formant ainsi, une fois placé sur un membre traumatisé, une contention orthopédique rigide, adoptant parfaitement les contours dudit membre, lorsque ladite résine de polyuréthane est recristallisée.

Fig. 3

EP 1 656 916 A1

**Description**

**Objet de l'invention**

La présente invention concerne le domaine des contentions orthopédiques. Elle se rapporte plus particulièrement à un élément tubulaire préétirable pour la réalisation d'une contention orthopédique rigide ou semi-rigide qui a pour effet de limiter les mouvements possibles du membre traumatisé.

**Introduction et problématique**

Actuellement une proportion importante de patients consultant les services d'urgence des hôpitaux bénéficie de la mise en place d'une contention orthopédique, par exemple pour les traumatismes ostéo-articulaires des membres.

Lors du traitement d'un traumatisé, les contentions circulaires sont mises en place en enroulant progressivement des bandes préparées à l'avance autour du membre à soigner. Idéalement, la contention doit être parfaitement adaptée en offrant peu de possibilités de déplacement du membre immobilisé. Elle doit être confortable, peu encombrante, légère et esthétique. Elle doit par ailleurs être aisée à façonner, rapide et propre à placer par le praticien et également facile à enlever. Le matériau doit aussi être suffisamment durable, résistant à l'humidité, radio-transparent, non inflammable et non toxique.

Ces contentions sont généralement réalisées à partir de bandes plâtrées ou de bandes de résine qui ont la propriété de durcir dans un certain délai après les avoir trempées dans l'eau froide ou l'eau chaude selon les cas.

Sur un membre traumatisé, l'oedème habituel, s'installant dans les premières heures après le traumatisme, entraîne une augmentation importante des pressions sous l'immobilisation inextensible, obligeant souvent le clinicien à fendre la contention.

Un bon maintien, en l'absence de jeu, n'est toutefois possible que si une légère pression est exercée par la contention sur le membre, ce qui s'obtient en appliquant une certaine tension sur la bande pendant son enroulement en couches concentriques successives. Or le durcissement s'accompagne d'un retrait et toutes les portions de la contention ne durcissent pas en même temps. Il en résulte des inégalités de pressions exercées par la contention une fois solidifiée et des effets divers comme ceux catalogués sous le vocable d'algodystrophie.

On s'efforce communément de pallier cet inconvénient en interposant une ouate ou une matière de rembourrage. Dans ce cas cependant, une tension supérieure de la bande est nécessaire pour assurer le maintien du membre par compression de ladite matière de rembourrage et les inégalités de pression sur le membre restent aléatoires.

**Etat de la technique**

Durant des décennies, le plâtre était le seul matériau utilisé, ensuite, des contentions orthopédiques à base de résines polymérisables ont vu le jour. Néanmoins, et hormis une série d'autres inconvénients, tels que le poids pour le plâtre et la toxicité pour les résines thermodurcissables, la réaction exothermique du durcissement du plâtre ou de la polymérisation des résines expose le patient à un risque de brûlure important.

Certaines résines thermodurcissables sont parfois combinées avec des fibres de verre imprégnant un support tramé. Les fibres de verre présentent une série d'autres inconvénients intrinsèques et sont inadaptées au travail en milieu hospitalier.

Durant ces dernières années, on a mis au point des résines thermoplastiques possédant des seuils de fusion se situant dans des plages à la fois supérieures à la température du corps (37°C) et encore malléables à des températures inférieures auxquelles des brûlures sur le membre traumatisé pourraient être provoquées. Dans ce type de polymères, les plages de fusion sont généralement assez larges (50 à 70°C) et la recristallisation en dessous de ces températures (vers 40°C) est relativement lente, c'est à dire de l'ordre de 5 à 10 minutes. Les polyisoprènes et les polycaprolactones entrent dans cette catégorie de produits.

Les caractéristiques physiques quantifiables sur des matériaux utilisés pour des contentions orthopédiques sont généralement le module d'élasticité, la résistance à la rupture, à la fatigue et à l'usure.

Les matériaux thermoplastiques, qui se moulent sous l'action de la chaleur sont principalement utilisés pour traiter les traumatismes du poignet, de la main et des doigts. Selon leur composition chimique, ils auront un comportement plus caoutchouteux (polyisoprène) ou plus plastique (polyester, polycaprolactone)

La demande de brevet EP 1230937 A1, divulgue des compositions de polyuréthanne thermoplastique pour des applications orthopédiques à base d'un tissu de support flexible imprégné d'une résine semi-cristalline comprenant des nanocharges. L'objectif de cette demande de brevet est de réaliser des bandes imprégnées malléables avec une bonne adhésion sur elles-mêmes. La seule mise en température permettant l'utilisation de ces bandes sans risque de brûlure pour le patient.

Certains documents divulguent des produits imprégnés sous forme de feuilles ou de bandelettes pour le même domaine d'application. D'autres décrivent des matériaux de moulage *(casting material)* fait à partir de deux tissus, ou d'un tissu replié. Nous citerons pour exemple les documents US 4, 968, 542 ; US 6,077,240 *;* US 6,100,206 et US 6,595,938.

Le document US 3,656,475 décrit un procédé d'application de contentions tubulaires imprégnées sur un membre traumatisé. La polymérisation se fait ici au moment même de l'application de la contention avec tous les inconvénients que cela engendre, réaction exothermique, toxicité, irritation de la peau, etc.

### Buts de l'invention

La présente invention vise à fournir un élément tubulaire pré-étirable pour la réalisation d'une contention orthopédique n'ayant pas les inconvénients de l'état de la technique et permettant d'améliorer et de faciliter la pose sur un membre traumatisé.

### Résumé de l'invention

La présente invention divulgue un élément tubulaire pour la réalisation d'une contention orthopédique, comportant au moins un textile élastique servant de support d'imprégnation à un produit d'imprégnation, ledit produit d'imprégnation comportant une résine polyuréthanne, caractérisé en ce que ledit élément tubulaire est essentiellement étirable et rétractable de manière réversible par retour élastique dudit support d'imprégnation, dans un plan perpendiculaire (y, z) à l'axe longitudinal (x) dudit élément tubulaire lorsque ladite résine de polyuréthane est à une température supérieure à sa température de ramollissement et avant recristallisation, formant ainsi, une fois placé sur un membre traumatisé, une contention orthopédique rigide, adoptant parfaitement les contours dudit membre, lorsque ladite résine de polyuréthanne est recristallisée.

Selon des formes d'exécution particulières, l'invention comporte l'une ou plusieurs des caractéristiques suivantes :

- ladite résine de polyuréthane est une résine de polycaprolactone. ;
- ladite résine de polycaprolactone est une résine de polycaprolactone diol d'une masse moléculaire moyenne supérieure à 3000 Dalton ;
- ledit élément tubulaire possède à l'état recristallisé un module d'élasticité, dans le plan perpendiculaire (y,z) à l'axe longitudinal (x) dudit élément tubulaire, compris entre 10 et 20 Mpa ;
- ledit élément tubulaire possède à l'état recristallisé, un module d'élasticité dans la direction de l'axe longitudinal (x), compris entre 100 et 200 Mpa ;
- ledit textile élastique (1) comprend un tissu muni de moyens élastiques (2) permettant un retour élastique sur un membre traumatisé ;
- ledit textile élastique (1) a une élasticité environ 10 fois supérieure dans le plan (y,z) que dans la direction (x) ;
- lesdits moyens élastiques (2) sont des fibres à caractère élastomère ;
- ladite température de ramollissement de la résine est inférieure à 70°C et supérieure à 40°C ;
- ladite recristallisation a lieu en moins de 10 minutes à 20 °C ;
- ladite résine de polyuréthane recristallisée représente au moins 60% en poids dudit élément tubulaire.

L'invention divulgue par ailleurs un procédé de préparation d'un élément tubulaire selon la revendication 1 pour la réalisation d'une contention orthopédique comportant les étapes successives suivantes :

- imprégnation d'un support textile élastique tubulaire par une résine de polyuréthanne ;
- ramollissement de ladite résine par la chaleur ;
- étirage dudit élément tubulaire dans un plan perpendiculaire (y,z) à l'axe longitudinal (x) dudit tube pour le rendre apte à être posé sur un membre traumatisé.

L'invention précise par ailleurs que dans le procédé de préparation d'un élément tubulaire ladite résine de polyuréthanne est :

- une résine de polycaprolactone ;
- que ladite résine de polycaprolactone est une résine de polycaprolactone diol avec un poids moléculaire moyen supérieur à 3000 Dalton.

**Brève description des figures**

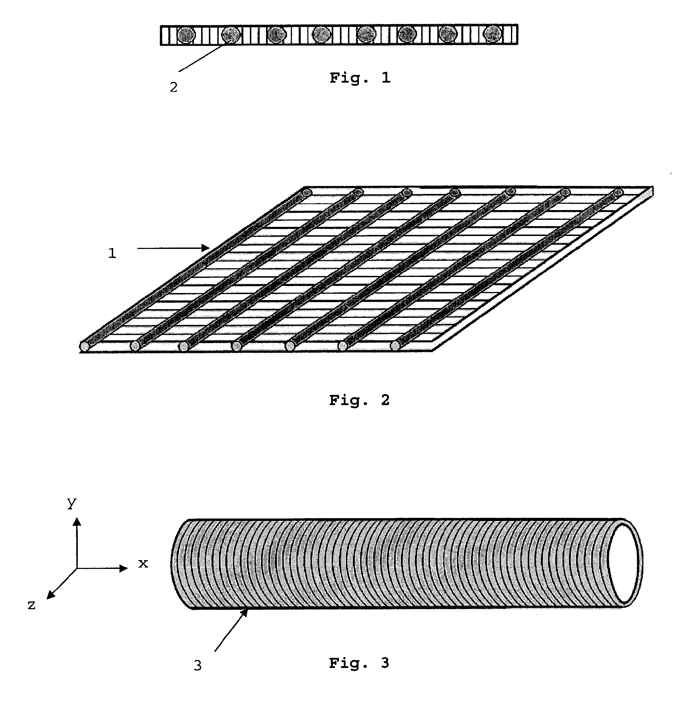

La figure 1 représente une vue de profil d'une partie du support textile de haute élasticité de la présente invention avec des fibres élastiques à distances régulières.

La figure 2 représente une vue en trois dimensions d'une partie du support textile de la présente invention.

La figure 3 représente l'élément tubulaire crée à partir du support textile élastique imprégné de résine

La figure 4 représente un bain d'eau chaude permettant de ramollir la résine d'imprégnation du support textile élastique formant l'élément tubulaire.

La figure 5 représente un cadre constituant une aide pour étirer l'élément tubulaire dans un plan transversal à l'axe longitudinal de cet élément

La figure 6 représente l'application de l'élément tubulaire étiré sur le cadre autour du membre traumatisé à immobiliser.

La figure 7 représente l'application de l'élément tubulaire étiré sur le cadre autour du membre traumatisé

La figure 8 représente la contention qui s'est formée par rétraction de l'élément tubulaire autour du membre traumatisé et qui s'est durcie en se refroidissant. La contention a parfaitement adopté la forme du membre.

La figure 9 représente schématiquement le dispositif permettant de mesurer le module de flexibilité du support textile flexible imprégné dans l'axe y et z.

La figure 10 représente le dispositif de la figure 9 en coupe et les différentes grandeurs mesurées sur les échantillons dans l'axe y et z.

La figure 11 représente le dispositif de mesure pour la détermination du module de flexibilité dans le sens longitudinal (x) de l'élément tubulaire sur les échantillons.

La figure 12 représente un schéma du procédé d'imprégnation pour la fabrication des éléments tubulaires de la présente invention.

**Description détaillée de l'invention**

La présente invention divulgue un élément tubulaire à géométrie cylindrique ou tronconique (non représenté) et préétirable pour la réalisation d'une contention orthopédique dont la longueur et le diamètre sont choisis pour s'adapter au membre à immobiliser.

L'élément tubulaire est essentiellement constitué d'un substrat élastique tubulaire, enduit ou imprégné d'un polymère thermoplastique recristallisé présentant une plage de fusion se situant en dessous de 70°C et de préférence entre 45 et 65°C.

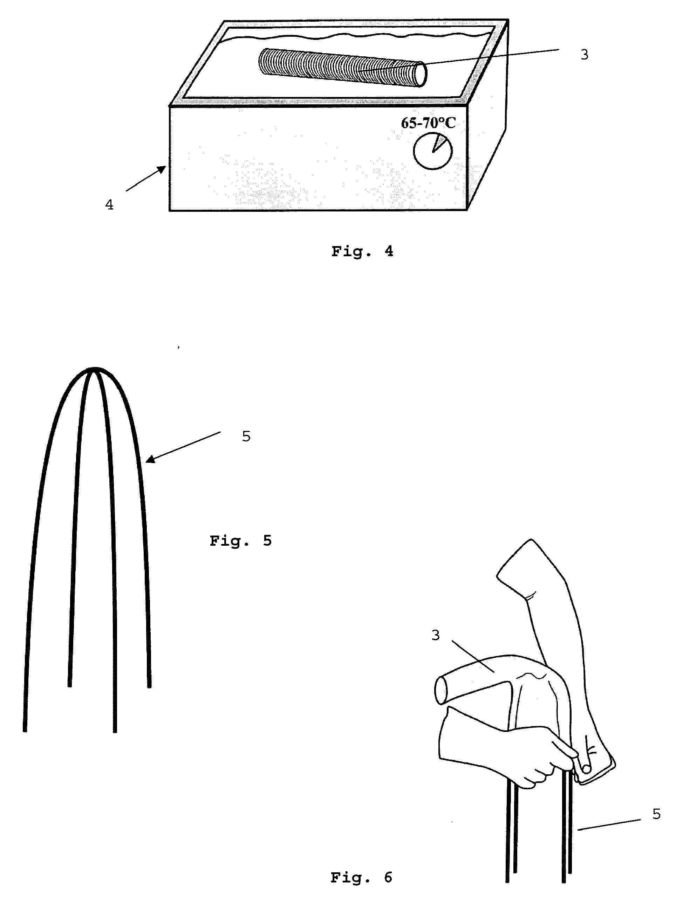

Pour la réalisation de la contention orthopédique, l'élément tubulaire est préparé en chauffant le substrat élastique imprégné dudit polymère thermoplastique à une température supérieure à 65°C. Ceci peut se faire par exemple en le trempant dans de l'eau chaude (>65°C) pendant quelques minutes ( 2 à 3 min), en vue de son application comme montré dans la figure 4.

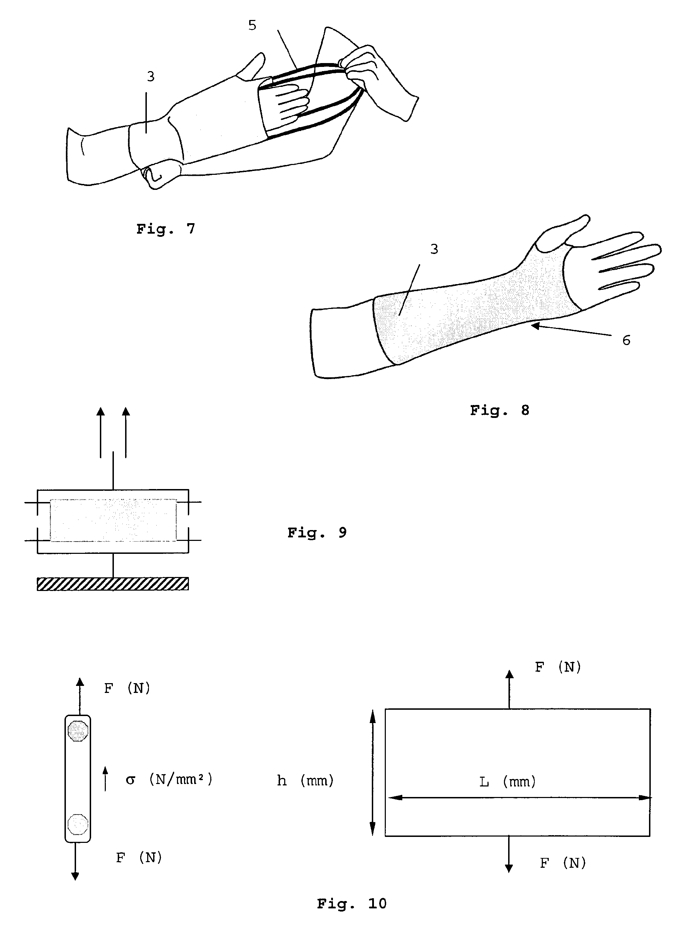

Une fois chauffé, l'élément tubulaire est alors étiré dans la direction transversale à l'axe longitudinal de l'élément tubulaire et passé sur le membre en l'enveloppant puis abandonné à couvrir librement le membre par l'effet du retour élastique du substrat avant de durcir une fois mis en place. Ceci est représenté dans les figures 6, 7 et 8. On peut aussi partir d'éléments pré-étirés (non représentés) dans ladite direction transversale et les placer sur un cadre rigide (cage d'écureuil) avant de les ramollir à la chaleur et de les déposer ensuite sur le membre à immobiliser.

Il en résulte de façon surprenante que la pression exercée à la surface du membre traumatisé ne présente plus de gradients significatifs, même en l'absence de mousse de rembourrage, et en particulier aux endroits de dénivelés importants comme autour des malléoles ou d'autres proéminences osseuses.

Dans un mode d'exécution préféré de l'invention, on utilise une manchette d'un substrat textile à haute élasticité (1) dans les directions transversales (y et z) à l'axe longitudinal (x) de l'élément tubulaire (3) et à élasticité réduite dans l'axe (x) de ladite manchette. Cette manchette est imprégnée d'une composition de résines thermoplastiques à basse température de ramollissement, qui se ramollit entre environ 45 et 65°C. Des exemples de ces compositions sont décrits dans le brevet US 4,316,457 et le brevet européen EP 0 659 087 B1.

La réalisation d'une contention sur un membre peut se faire en appliquant un ou plusieurs (non représenté) éléments tubulaires (3) l'un au-dessus de l'autre selon la rigidité désirée pour la contention. Si on applique plusieurs éléments tubulaires de façon concentrique on peut les assembler préalablement à la pose ou les poser successivement sur le membre. Avant la pose proprement dite, une découpe aux ciseaux ou à l'emporte pièce peut être pratiquée à un endroit de l'élément tubulaire pour créer une fenêtre ou laisser un doigt libre par exemple. Une telle ouverture peut aussi être pratiquée à posteriori à l'aide de ciseaux adaptés. Après la pose, il est également possible de retoucher la manchette à l'aide d'une source de chaleur car le processus de ramollissement à la chaleur suivi du durcissement en se refroidissant (recristallisation) est réversible. Ceci peut se faire par exemple à l'aide d'un dispositif soufflant de l'air chaud.

En fin de traitement, l'ablation de la contention selon la présente invention peut se faire simplement à l'aide de ciseaux.

Dans le cadre de l'étude sur ces jerseys tubulaires, nous avons réalisé des essais de traction permettant de quantifier la combinaison de l'élasticité du jersey et celle de la résine. Ces essais permettent de déterminer la pression ( mm Hg ) exercée par le jersey imprégné sur le membre traumatisé, ce qui permet de déterminer et de cibler les formulations adéquates ainsi que le type de jersey à utiliser. Les propriétés de jersey sont fonction de sa largeur, du diamètre de l'élément tubulaire et du nombre de fils élastiques.

L'élément tubulaire 3 de la présente invention est réalisé par un procédé d'imprégnation. Avant cette étape, il est nécessaire de sélectionner le type de substrat à imprégner. Ce substrat de forme tubulaire sera choisi en fonction de la taille du membre à soigner. Il existe plusieurs textiles répondant aux caractéristiques d'élasticité, mais dans le mode d'exécution préféré de l'invention, on utilise du coton tissé incorporant des fibres élastiques 2, tel que du jersey, il existe différentes largeurs de jersey tubulaire.

Dans le cadre du développement de la présente invention, nous avons notamment utilisé des jerseys tubulaires de la société Spronken S.A. Les références de ces jerseys tubulaires sont les suivantes: jersey tubulaire coton + Lycra X4 comportant 1, 2 ou 4 élastiques par intervalle.

Dans un exemple de la présente invention, le substrat coton tubulaire non imprégné a un poids surfacique de 580g/m$^2$, après imprégnation ce poids passe à 2311g/m$^2$. Par différence nous obtenons simplement le poids surfacique de résine sèche, soit 1731g/m$^2$, ce qui représente une teneur en poids de résine sèche de 75%, pour un substrat avec quatre élastiques par intervalle et 7 cm de largeur à plat.

Le tableau 1, montre que les meilleurs résultats sont obtenus avec le jersey tubulaire comportant 4 élastiques par intervalle tissé. Celui-ci bénéficie d'un grand pouvoir de rappel et il permet donc une meilleur moulabilité sur le membre traumatisé.

Essais de traction selon l'axe y et z

Les essais de traction sur les jerseys tubulaires imprégnés et non imprégnés sont réalisés avec une machine de traction de marque WOLPERT TT 1220 10kN (1987) dans un bac d'eau chaude à une température de 65°C. Le dispositif de traction comprend deux tiges d'acier parallèles d'une section de 10 mm solidaires des mords fixe et mobile respectivement (voir figure 9 et 10).

Le jersey est enfilé sur les tiges métalliques sur une longueur de 200 mm. La vitesse de traction est de 1m/min. La vitesse d'avance du papier de l'enregistreur potentiométrique est de 60cm/min. De cette façon, il est possible d'obtenir une force pour un allongement donné. A partir de cet essai de traction à plat, nous avons transposé la force donnant un h donné en une pression en mm Hg du cylindre correspondant.

La contrainte σ (N/mm$^2$) pour l'essai de traction à plat s'écrit:

$$\sigma = F / (2*L*e) \quad (\text{équation 1})$$

e : épaisseur du jersey tubulaire
L : longueur de l'élément tubulaire testé

En service, le jersey est de forme tubulaire, il est donc possible de lui appliquer la formule des chaudières pour déterminer la pression qu'il exerce sur le membre d'un rayon r donné.

$$\sigma = P * r / e \quad (\text{équation 2})$$

En remplaçant (l'équation 2) dans (l'équation 1), on obtient :

$$\sigma = P * r / e = F / (2*L*e)$$

$$P = F / (2 * L * r) \quad (\text{équation } 3)$$

En remplaçant le rayon du jersey tubulaire r en fonction d'une valeur connue telle que la hauteur h utilisée pendant l'essai de traction:

$$2 * L * h = 2 * \pi * r * L$$

$$r = h / \pi$$

Finalement, l'équation (3) s'écrit:

$$P = F * \pi / (2 * L * h)$$

La pression calculée par cette équation est donnée en N/mm$^2$ (MPa), en mm de mercure (mm Hg) cela donne :

$$P(mmHg) = P (Pa) * (760/101325)$$

Le tableau ci-dessous, nous permet de visualiser les résultats pour les différents types de jerseys tubulaires et les différentes formulations.

Comme attendu, plus le nombre d'élastiques dans l'armure du jersey augmente et plus la force augmente pour un même allongement.

Il y a également une différence de pression développée entre les échantillons contenant du HDI (hexaméthylène diisocyanate) ou du TDI (tolylène diisocyanate) celle-ci est environ deux fois plus élevée pour les formulations avec HDI. Ceci montre que la pression peut être modulée en adaptant la formulation de la résine ainsi que le nombre d'élastiques. Par exemple, une combinaison de CAPA 240 (polycaprolactone diol 240 (MW : 4280 g/mole) HDI et le jersey 4 élastiques (7cm) extrapolée à partir des résultats du tableau 1, donne les meilleurs résultats. Il s'agit d'un bon compromis pour le placement sur le cadre et le temps de cristallisation pour le patient. La pression exercée pour le patient est également acceptable et il assure un bon maintien du membre.

*Tableau* 1: *Essai* de *traction transposé en mesure* de *pression*

| Référence | Nbres | Largeur au repos élastiq à plat | Formulation | | | | T °C eau eau | Pression du jersey en fonction de l'allongement (mmHg) | | | |
|---|---|---|---|---|---|---|---|---|---|---|---|
| | | | CAPA 240 | HDI | TDI | stoech ISO/OH | | Allongement % | | | |
| | | (cm) | g | g | g | (-) | | 50% | 100% | 150% | 200% |
| 1 élast 6cm | 1 | 6 | Non imprégné | | | | | 3,93 | 6,87 | 9,43 | 12,27 |
| 230403/2 | 1 | 6 | 100 | 3.9 | | 1,00 | 63,6 | 7,53 | 8,10 | 9,23 | 11,29 |
| 1 élast 7cm | 1 | 7 | Non imprégné | | | | | 2,52 | 3,58 | 4,21 | 5,89 |
| 130603/1A1 | 1 | 7 | 100 | | 4.04 | 1,00 | 65,5 | 3,93 | 4,63 | 5,05 | 5,89 |

Suite de tableau

|           |   | (cm) | g   | g   | g   | (-)  |      | 50%  | 100%  | 150%  | 200%  |
|-----------|---|------|-----|-----|-----|------|------|------|-------|-------|-------|
| 130503/6-b | 2 | 7   | 100 |     | 4.1 | 1,02 | 64,8 | 7,29 | 7,99  | 8,50  | 9,33  |
| 130503/7-a | 2 | 7   | 100 |     | 4.2 | 1,05 | 64,5 | 7,85 | 8,31  | 9,43  | 10,66 |
| 4 élast 7cm | 4 | 7   | Non imprégné |     |     |      |      | 8,28 | 9,89  | 10,94 | 12,20 |
| 230403/1  | 4 | 7   | 100 | 3.9 |     | 1,00 | 65,6 | 9,82 | 11,57 | 10,52 | 11,92 |

Pour la détermination du module d'élasticité du jersey tubulaire imprégné, nous avons réalisé l'essai de traction à une température de 20°C selon la méthode expliquée plus haut. Nous obtenons un graphique de la force en fonction du déplacement. A partir de ces données nous déterminons le module d'élasticité comme suit :

Essai de traction: échantillon 230403/1

Une déformation ($\Delta h/h = \varepsilon$) de 6.42% correspond une force de 570N.

Nous déterminons ensuite la contrainte (N/mm$^2$) :

$$\sigma = F / ( 2*L*e)$$

$$\sigma = 0.7125 \ N/mm^2$$

Le module d'élasticité est donc égal à 11 N/mm$^2$ soit 11 MPa.

Le module d'élasticité suivant y ou z se situe donc dans une zone de approximativement 11 MPa.

Mesure du module d'élasticité dans le sens longitudinal (selon x)

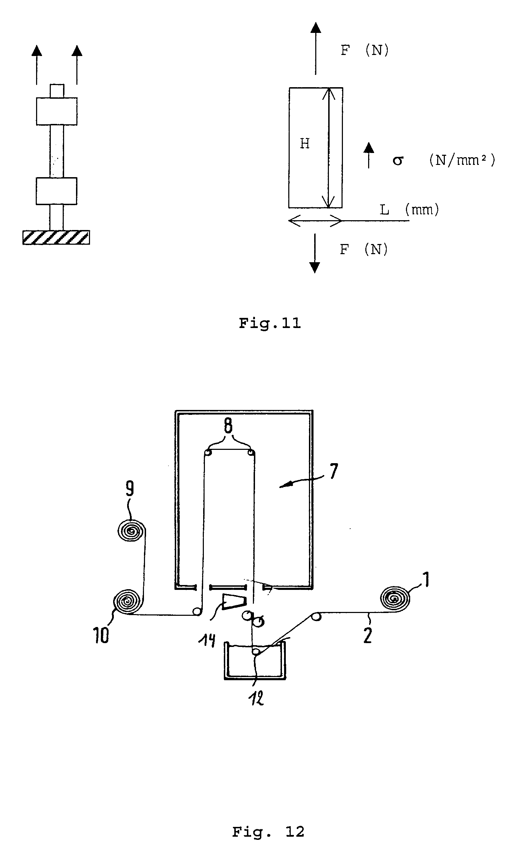

La détermination du module d'élasticité pour l'axe longitudinal s'obtient par le dispositif suivant (fig 12 et 13). Nous avons utilisé une machine de traction de type WOLPERT TT1220 10kN à température de 20°C. Le jersey tubulaire imprégné d'une longueur de 200 mm est maintenu entre deux mords de 45 mm de hauteur. La hauteur utile de l'échantillon est de 110mm. La contrainte σ (N/mm$^2$) pour l'essai de traction à plat dans le sens longitudinal s'écrit :

$$\sigma = F/ (2*L*e) \quad (\text{équation 1})$$

e: épaisseur du jersey tubulaire
L : largeur de l'élément tubulaire

Essai de traction :

Une déformation ($\Delta H/H$) de 3.33% correspond une force de 1575 N.

Nous déterminons la contrainte par l'équation 1, σ = 5,625 N/mm$^2$

Le module d'élasticité est donc égal à 168 N/mm$^2$ soit 168 MPa. Ce module est environ 10 fois plus élevé que le module d'élasticité dans le sens transversal. Cela semble compréhensible étant donné la très faible élasticité du jersey tubulaire dans le sens longitudinal.

Le procédé d'imprégnation du substrat textile élastique peut se décrire de la façon suivante (voir Fig. 12) :

Un rouleau de 25m de substrat textile tubulaire 1, est déposé en bas d'une tour d'imprégnation sur un arbre permettant la rotation de ce rouleau. Le jersey se déroule grâce à une machine de traction 10 se situant de l'autre côté de l'installation. Le jersey passe dans un bac d'imprégnation 12, thermostatisé, contenant au moins une résine, au moins un solvant, au moins un catalyseur et éventuellement un pigment. Le jersey imprégné entame une montée dans un caisson chauffé 7 de la tour d'imprégnation. Cette vitesse de montée engendrée par la machine de traction 10 est variable, et est ajustée de sorte que le temps de séjour du jersey imprégné dans le caisson chauffé permet l'achèvement de la polymérisation et l'évaporation du solvant. Le tissu ainsi imprégné est amené, grâce à des cylindres de renvoi 8, vers la sortie pour être stocké dans des ensouples.

Après avoir effectué un contrôle qualité de l'imprégnation (DMTA et rhéologie de la résine), le jersey tubulaire va être façonné pièce par pièce. Disposant d'une longueur de 25m, il est nécessaire de les couper à des longueurs définies pour le conditionnement. Le jersey tubulaire disposé sur les ensouples après le processus d'imprégnation se trouve alors à plat (écrasé) et les parties intérieures du jersey tubulaire ont tendance à coller entre elles. Il est dès lors obligatoire d'ouvrir le jersey tubulaire pour la préparation au conditionnement. Pour ce faire, le jersey imprégné est coupé à ± 1m de longueur. Il est plongé dans un bain thermostatisé à 65°C pendant deux à trois minutes afin que la résine soit à l'état fondu. Le jersey est ensuite retiré du bain et un des côtés du jersey est obturé par exemple par simple pression manuelle. Le côté libre va contenir un moyen pour gonfler la contention tubulaire comme par exemple un tuyau d'air comprimé. Une fois ce dispositif mis en place, l'air comprimé est envoyé à l'intérieur du jersey, celui-ci s'écarte sous l'effet de la pression et forme un tube de diamètre nominal équivalent au diamètre du jersey de départ. L'application de l'air comprimé permet également de diminuer la température du jersey et de recristalliser la résine. Le jersey conservera alors sa forme tubulaire et sera prêt à l'emploi.

Le tube d'un mètre de long pourra être découpé en sous-longueurs à l'aide de ciseaux. Des morceaux seront par exemple ajustés à 45 cm. L'étape suivante est facultative pour le conditionnement, elle consiste à écarter le jersey pour faciliter le travail du praticien lors de la pose de la contention. Nous replaçons le jersey imprégné dans le bain thermostatisé à 65°C pendant deux minutes. Le jersey est ensuite disposé sur l'applicateur de jersey tubulaire (en forme de cage d'écureuil) en exerçant la pression nécessaire. Cette étape réalisée, l'entièreté du dispositif est passé sous l'eau froide pendant deux minutes afin que la résine cristallise. Le durcissement de la résine étant obtenu, il est possible de faire glisser et d'extraire le jersey de l'applicateur. Le jersey n'a plus l'aspect d'un tube mais bien d'un tuyau carré de plus grande dimension. Ce carré peut dès lors être conditionné à plat par simple pression. Dans cette configuration il prend très peu de place. Lorsque l'élément tubulaire est conditionné de cette façon, lors de la réception du produit, le praticien place le jersey préétiré sur l'applicateur. Une découpe à l'aide d'un ciseau ou à l'aide d'un emporte pièce peut être réalisée pour laisser un doigt libre si nécessaire. Ensuite, il plonge le dispositif dans un bain thermostatisé à 65°C pendant deux minutes. Pendant ce temps, le praticien place un jersey de protection non imprégné sur le membre à traiter. L'élément tubulaire, une fois enlevé du bain chaud commence déjà à se refroidir et est alors passé à une température d'environ 40°C sur le membre en l'enveloppant puis abandonné à couvrir librement le membre par l'effet du retour élastique du substrat avant de durcir une fois mis en place. La recristallisation mettant plusieurs minutes, la contention reste encore malléable même à une température de 35°C.

En fonction du traumatisme à traiter, le praticien applique une ou plusieurs couches de jersey tubulaire sur le membre. Le praticien réalise ensuite les finitions, c'est à dire la découpe du pouce et les extrémités de la contention. Le praticien immobilise le membre en exerçant une légère pression pendant 3 à 4 minutes. Après une dizaine de minutes la contention atteint un seuil acceptable de rigidité et l'immobilisation du membre à traiter est assurée.

En fin de traitement, l'ablation de la contention réalisé avec l'élément tubulaire de la présente invention peut se faire simplement à l'aide de ciseaux.

L'avantage de la présente invention est qu'elle permet un gain de temps considérable par rapport au bandes imprégnées, à enrouler, connues dans l'état de la technique. La pose de la contention tubulaire se fait en une seule fois, il n'y a aucune réaction chimique exothermique provoquant un dégagement de chaleur ou de produits toxiques. La contention réalisée à partir de l'élément tubulaire de la présente invention est radio-transparente et légère, elle résiste à l'eau (piscine) et peut être fabriquée dans une couleur proche de celle de la peau. Des parties pouvant éventuellement présenter certains inconforts ou des pressions indésirables sur le membre traumatisé peuvent être remodelées à l'aide de la chaleur.

Par ailleurs, étant donné la facilité de pose, la contention peut être renouvelée très facilement et aussi souvent que nécessaire.

**Légende**

1. Substrat textile à haute élasticité
2. Elastiques incorporés dans le tissu
3. Elément tubulaire réalisé à partir du tissu Jersey. Les élastiques se trouvant dans un plan perpendiculaire à l'axe longitudinal du tube
4. Réservoir d'eau chaude à 65-70°C, nécessaire pour ramollir la résine qui imprègne le tissu Jersey
5. Cadre rigide
6. Contention réalisée sur membre traumatisé
7. Chauffage de la tour
8. Cylindre de renvoi
9. Bobine de récupération
10. Machine de traction
11. Tringle
12. Bac d'imprégnation
13. Cylindre d'essorage
14. Soufflette

**Revendications**

1. Elément tubulaire (3) pour la réalisation d'une contention orthopédique (6), comportant au moins un textile élastique (1) servant de support d'imprégnation à un produit d'imprégnation, ledit produit d'imprégnation comportant une résine polyuréthanne, **caractérisé en ce que** ledit élément tubulaire (3) est essentiellement étirable et rétractable de manière réversible par retour élastique dudit support d'imprégnation, dans un plan perpendiculaire (y,z) à l'axe longitudinal (x) dudit élément tubulaire (3) lorsque ladite résine de polyuréthane est à une température supérieure à sa température de ramollissement et avant recristallisation, formant ainsi, une fois placé sur un membre traumatisé, une contention orthopédique rigide (6), adoptant parfaitement les contours dudit membre, lorsque ladite résine de polyuréthanne est recristallisée.

2. Elément tubulaire (3) selon la revendication 1 **caractérisé en ce que** ladite résine de polyuréthane est une résine de polycaprolactone.

3. Elément tubulaire (3) selon la revendication 2 **caractérisé en ce que** ladite résine de polycaprolactone est une résine de polycaprolactone diol d'une masse moléculaire moyenne supérieure à 3000 Dalton.

4. Elément tubulaire (3) selon la revendication 1 **caractérisé en ce que** ledit élément possède à l'état recristallisé un module d'élasticité, dans le plan perpendiculaire (y, z) à l'axe longitudinal (x) dudit élément tubulaire, compris entre 10 et 20 MPa.

5. Elément tubulaire selon la revendication 1 **caractérisé en ce qu'**il possède à l'état recristallisé, un module d'élasticité dans la direction de l'axe longitudinal (x), compris entre 100 et 200 MPa.

6. Elément tubulaire selon la revendication 1 **caractérisé en ce que** ledit textile élastique (1) comprend un tissu muni de moyens élastiques (2) permettant un retour élastique sur un membre traumatisé.

7. Elément tubulaire selon la revendication 6 **caractérisé en ce que** ledit textile élastique (1) a une élasticité environ 10 fois supérieure dans le plan (y, z) que dans la direction (x).

8. Elément tubulaire selon la revendication 6, **caractérisée en ce que** lesdits moyens élastiques (2) sont des fibres à caractère élastomère.

9. Elément tubulaire selon la revendication 1 **caractérisé en ce que** ladite température de ramollissement de la résine est inférieure à 70°C et supérieure à 40°C.

10. Elément tubulaire selon la revendication 1 **caractérisé en ce que** ladite recristallisation a lieu en moins de 10 minutes à 20 °C.

11. Elément tubulaire selon la revendication 1 **caractérisé en ce que** ladite résine de polyuréthane recristallisée représente au moins 60% en poids dudit élément tubulaire.

12. Procédé de préparation d'un élément tubulaire (3) selon la revendication 1 pour la réalisation d'une contention orthopédique (6) comportant les étapes successives suivantes :

- imprégnation d'un support textile élastique tubulaire par une résine de polyuréthanne ;
- ramollissement de ladite résine par la chaleur ;
- étirage dudit élément tubulaire dans un plan perpendiculaire (y, z) à l'axe longitudinal (x) dudit tube pour le rendre apte à être posé sur un membre traumatisé.

13. Procédé de préparation d'un élément tubulaire (3) selon la revendication 12 **caractérisé en ce que** ladite résine de polyuréthanne est une résine de polycaprolactone.

14. Procédé de préparation d'un élément tubulaire (3) selon la revendication 12 **caractérisé en ce que** ladite résine de polycaprolactone est une résine de polycaprolactone diol avec un poids moléculaire moyen supérieur à 3000 Dalton.

15. (Pour USA uniquement) Procédé d'application de l'élément tubulaire de la revendication 1, pour la réalisation d'une contention orthopédique comprenant les étapes successives suivantes :

- imprégnation d'un élément tubulaire d'un support textile élastique par une résine de polycaprolactone ;
- ramollissement de ladite résine par la chaleur et étirage dudit élément tubulaire dans un plan perpendiculaire (z, y) à l'axe longitudinal (x) dudit tube ;
- présentation dudit élément tubulaire étiré autour du membre traumatisé ;
- rétraction dudit élément tubulaire étiré autour du membre traumatisé ;
- refroidissement dudit élément tubulaire étiré autour du membre traumatisé pour former une contention orthopédique.

2

Fig. 1

1

Fig. 2

Y

X

z

3

Fig. 3

Fig. 4

Fig. 5

Fig. 6

Fig. 7

Fig. 8

Fig. 9

F (N)

σ (N/mm²)

F (N)

h (mm)

F (N)

L (mm)

F (N)

Fig. 10

13

F (N)

H

σ (N/mm²)

L (mm)

F (N)

Fig.11

Fig. 12

**Office européen des brevets**

**RAPPORT DE RECHERCHE EUROPEENNE**

Numéro de la demande

EP 04 44 7247

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| X | US 5 514 080 A (BLOTT ET AL)<br>7 mai 1996 (1996-05-07)<br>* colonne 2, ligne 50-58 *<br>* colonne 3, ligne 41-50; exemple 1 *<br>* colonne 10, ligne 36 - colonne 11, ligne 30 * | 1 | A61F13/04<br>A61F13/06<br>A61F13/10<br>A61F13/08 |
| A | ----- | 2-15 | |
| X | US 4 940 047 A (RICHTER ET AL)<br>10 juillet 1990 (1990-07-10)<br>* colonne 2, ligne 18-20 *<br>* colonne 6, ligne 29 - colonne 7, ligne 54; revendication 10 * | 1 | |
| A | ----- | 2-15 | |
| D,X | US 3 656 475 A (JAMES R. HANRAHAN JR)<br>18 avril 1972 (1972-04-18)<br>* colonne 4, ligne 4-59; revendication 1 *<br>----- | 1 | |
| A | US 6 544 204 B1 (BRANDHOFF STEFAN)<br>8 avril 2003 (2003-04-08)<br>* colonne 2, ligne 30-48 *<br>----- | 1-15 | **DOMAINES TECHNIQUES RECHERCHES (Int.Cl.7)**<br><br>A61F |
| A | WO 01/54639 A (SMITH & NEPHEW, INC)<br>2 août 2001 (2001-08-02)<br>* page 4, ligne 7-17 *<br>----- | 1-15 | |
| A | US 2004/077980 A1 (STESS RICHARD M ET AL)<br>22 avril 2004 (2004-04-22)<br>* alinéas [0050] - [0052] *<br>----- | 1-15 | |
| X | EP 0 326 285 A (SMITH & NEPHEW P.L.C)<br>2 août 1989 (1989-08-02)<br>* page 4, ligne 7-36; exemples 1,2 *<br>----- | 1 | |

Le présent rapport a été établi pour toutes les revendications

| Lieu de la recherche | Date d'achèvement de la recherche | Examinateur |
|---|---|---|
| Munich | 2 août 2005 | Lanniel, G |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

..................................................................

& : membre de la même famille, document correspondant

EPO FORM 1503 03.82 (P04C02)

## ANNEXE AU RAPPORT DE RECHERCHE EUROPEENNE
## RELATIF A LA DEMANDE DE BREVET EUROPEEN NO.

EP 04 44 7247

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

02-08-2005

| Document brevet cité au rapport de recherche | | Date de publication | Membre(s) de la famille de brevet(s) | | Date de publication |
|---|---|---|---|---|---|
| US 5514080 | A | 07-05-1996 | AT | 290839 T | 15-04-2005 |
| | | | AU | 644077 B2 | 02-12-1993 |
| | | | AU | 5740890 A | 18-12-1990 |
| | | | CA | 2044260 A1 | 19-11-1990 |
| | | | DE | 69034185 D1 | 21-04-2005 |
| | | | DE | 69034185 T2 | 28-07-2005 |
| | | | DK | 591130 T3 | 18-04-2005 |
| | | | EP | 0591130 A1 | 13-04-1994 |
| | | | WO | 9014060 A1 | 29-11-1990 |
| | | | GB | 2246956 A ,B | 19-02-1992 |
| | | | JP | 5500759 T | 18-02-1993 |
| | | | JP | 3222460 B2 | 29-10-2001 |
| | | | ZA | 9003834 A | 26-06-1991 |
| US 4940047 | A | 10-07-1990 | DE | 3726268 A1 | 05-01-1989 |
| | | | AT | 93909 T | 15-09-1993 |
| | | | AU | 608622 B2 | 11-04-1991 |
| | | | AU | 1836988 A | 05-01-1989 |
| | | | BR | 8803090 A | 31-01-1989 |
| | | | CA | 1330917 C | 26-07-1994 |
| | | | CN | 1030269 A ,C | 11-01-1989 |
| | | | DD | 281837 A5 | 22-08-1990 |
| | | | DE | 3883660 D1 | 07-10-1993 |
| | | | DK | 345788 A | 25-12-1988 |
| | | | EP | 0301214 A2 | 01-02-1989 |
| | | | ES | 2042650 T3 | 16-12-1993 |
| | | | FI | 883007 A ,B, | 25-12-1988 |
| | | | HU | 50231 A2 | 28-12-1989 |
| | | | IE | 61729 B1 | 30-11-1994 |
| | | | IL | 86817 A | 16-08-1991 |
| | | | JP | 1014377 A | 18-01-1989 |
| | | | KR | 9609086 B1 | 10-07-1996 |
| | | | NO | 882524 A ,B, | 27-12-1988 |
| | | | PH | 26348 A | 29-04-1992 |
| | | | PT | 87787 A ,B | 31-05-1989 |
| | | | SU | 1600623 A3 | 15-10-1990 |
| | | | ZA | 8804482 A | 29-03-1989 |
| US 3656475 | A | 18-04-1972 | CA | 920462 A1 | 06-02-1973 |
| | | | DE | 2015534 A1 | 11-03-1971 |
| | | | GB | 1307942 A | 21-02-1973 |
| US 6544204 | B1 | 08-04-2003 | DE | 19841561 A1 | 07-10-1999 |
| | | | AT | 265237 T | 15-05-2004 |
| | | | AU | 743995 B2 | 14-02-2002 |
| | | | AU | 3332399 A | 25-10-1999 |

EPO FORM P0460

Pour tout renseignement concernant cette annexe : voir Journal Officiel de l'Office européen des brevets, No.12/82

**EP 1 656 916 A1**

**ANNEXE AU RAPPORT DE RECHERCHE EUROPEENNE
RELATIF A LA DEMANDE DE BREVET EUROPEEN NO.**  EP 04 44 7247

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

02-08-2005

| Document brevet cité au rapport de recherche | | Date de publication | Membre(s) de la famille de brevet(s) | | Date de publication |
|---|---|---|---|---|---|
| US 6544204 | B1 | | CA | 2326567 A1 | 14-10-1999 |
| | | | DE | 59909338 D1 | 03-06-2004 |
| | | | DK | 1066064 T3 | 28-06-2004 |
| | | | WO | 9951281 A1 | 14-10-1999 |
| | | | EP | 1066064 A1 | 10-01-2001 |
| | | | ES | 2220055 T3 | 01-12-2004 |
| | | | JP | 2002510532 T | 09-04-2002 |
| | | | NO | 20004834 A | 26-09-2000 |
| | | | PT | 1066064 T | 31-08-2004 |
| | | | SI | 1066064 T1 | 31-12-2004 |
| WO 0154639 | A | 02-08-2001 | AU | 2964501 A | 07-08-2001 |
| | | | EP | 1202695 A1 | 08-05-2002 |
| | | | JP | 2003524491 T | 19-08-2003 |
| | | | WO | 0154639 A1 | 02-08-2001 |
| US 2004077980 | A1 | 22-04-2004 | AUCUN | | |
| EP 0326285 | A | 02-08-1989 | CA | 1333870 C | 10-01-1995 |
| | | | AU | 615250 B2 | 26-09-1991 |
| | | | AU | 2871189 A | 27-07-1989 |
| | | | DE | 68908573 D1 | 30-09-1993 |
| | | | DE | 68908573 T2 | 23-12-1993 |
| | | | EP | 0326285 A2 | 02-08-1989 |
| | | | ES | 2045401 T3 | 16-01-1994 |
| | | | GB | 2214199 A ,B | 31-08-1989 |
| | | | JP | 2005944 A | 10-01-1990 |
| | | | JP | 2803826 B2 | 24-09-1998 |
| | | | US | 5403267 A | 04-04-1995 |
| | | | AT | 93378 T | 15-09-1993 |
| | | | ZA | 8900514 A | 27-09-1989 |

EPO FORM P0460

Pour tout renseignement concernant cette annexe : voir Journal Officiel de l'Office européen des brevets, No.12/82